# FASCICULE DE BREVET EUROPEEN

(11) **EP 1 336 391 B1**
(45) Date de publication et mention de la délivrance du brevet: **14.12.2011**
(21) Numéro de dépôt: 03290346.0
(22) Date de dépôt: 12.02.2003
(51) Int. Cl.: A61F 2/00

(54) **Plaque herniaire à organe de déploiement non permanent**
Hernienplatte zum nicht ständigen Einbringen
Hernia plate for non permanent deploying

(30) Priorité: 13.02.2002 FR 0201774
(43) Date de publication de la demande: 20.08.2003
(73) Titulaire: Cousin Biotech, 59117 Wervicq Sud (FR); Cabaniols, Henri, 34090 Montpellier (FR)
(72) Inventeur: Cabaniols, Henri Georges Francis, 34090 Montpellier (FR); Solecki, Gilles Francis, 59390 Lys lez Lannoy (FR)
(74) Mandataire: Thinat, Michel

(56) Documents cités:
- EP-A- 0 557 964
- US-A- 5 122 155
- US-A- 5 176 692
- US-A- 5 258 000
- US-A- 5 366 460
- US-A- 5 540 711

## Description

La présente invention concerne, de façon générale, le domaine des implants chirurgicaux.

Plus précisément, l'invention concerne une plaque herniaire du type de celles qui comprennent une poche formée de première et seconde couches textiles liées l'une à l'autre par leurs périphéries respectives, et des moyens d'expansion assurant un déploiement de la poche derrière la face interne d'une paroi musculaire.

Une plaque de ce type est décrite dans le document de brevet EP 0 252 607.

L'implant décrit dans ce document antérieur prend la forme d'un coussin rempli de fibres destinées à être résorbées par le corps humain.

Non seulement cet implant se trouve ainsi gonflé en permanence, de sorte que son insertion dans un orifice herniaire est délicate, mais il représente un corps étranger de volume important que l'organisme du patient dans lequel il est implanté doit résorber.

Une autre plaque herniaire est connue du document EP-A-0 557 964.

Dans ce contexte, la présente invention a pour but de proposer une plaque herniaire offrant une grande facilité d'insertion et perturbant de façon minimale l'organisme dans lequel elle est implantée.

A cette fin, la plaque herniaire de l'invention, par ailleurs conforme à la définition générique qu'en donne le préambule ci-dessus, est essentiellement caractérisée en ce que les moyens d'expansion sont reçus de façon amovible dans la poche, en ce que la première couche textile présente un orifice permettant le retrait des moyens d'expansion hors de la poche après le déploiement de celle-ci, et en ce que les moyens d'expansion passent par déformation réversible d'une configuration déployée à une configuration ramassée en boule, ce dont il résulte que la plaque dans son ensemble peut elle-même adopter une configuration en boule pour permettre son insertion dans le canal herniaire.

La seconde couche textile est par exemple conformée en disque.

L'orifice peut alors avoir une position centrale par rapport à la poche et la première couche textile peut être découpée en plusieurs secteurs radiaux distribués autour de l'orifice et se recouvrant mutuellement. Les secteurs radiaux peuvent être fixés par un lien coulissant circulaire.

Dans un premier mode de réalisation possible de l'invention, les moyens d'expansion comprennent un ballonnet étanche au gaz et / ou à un liquide éventuellement radio-opaque, ce ballonnet comprenant un conduit plus ou moins rigide de gonflage, traversant l'orifice de la première couche. Le liquide plus ou moins radio-opaque permet un contrôle radioscopique du bon positionnement de la plaque.

Le ballonnet, en configuration déployée, peut s'étendre dans un plan médian et être conformé en étoile, en fleur, en spirale, en serpentin, en grille, en matelas, en roue, ou en tore simple ou multiple. La matière constituant le ballonnet peut être plus ou moins élastique ou rigide.

En variante, ce ballonnet peut aussi s'étendre en volume et être conformé en hélice ou en accordéon dans sa configuration déployée.

Dans un second mode de réalisation possible de l'invention, les moyens d'expansion peuvent comprendre un fil ressort, par exemple un fil métallique monofilament.

Ce fil ressort, en configuration déployée, est avantageusement conformé en étoile, en fleur, en spirale, en serpentin, ou en huit simple ou multiple.

Par ailleurs, le fil ressort est de préférence rendu extractible de la poche par traction sur un brin du fil ressort accessible à travers l'orifice.

Enfin, quel que soit son mode de réalisation, la plaque herniaire de l'invention peut comprendre des fils de suture solidaires de la première couche textile.

D'autres caractéristiques et avantages de l'invention ressortiront clairement de la description qui en est faite ci-après, à titre indicatif et nullement limitatif, en référence aux dessins annexés, dans lesquels:
- la Figure 1 est une vue en coupe d'une région herniaire, susceptible d'être traitée par insertion de la plaque de l'invention;
- la Figure 2A est une vue en coupe d'une plaque conforme à un premier mode de réalisation de l'invention;
- la Figure 2B est une vue de face de la plaque herniaire illustrée à la figure 2A;
- la Figure 3A est une vue en coupe d'une plaque conforme à une variante du premier mode de réalisation de l'invention;
- la Figure 3B est une vue de face agrandie de la plaque herniaire illustrée à la figure 3A;
- la Figure 4A est une vue en coupe d'une plaque semblable à celle qu'illustre la figure 2A, équipée de fils de suture traversant une couche de la prothèse et sertis à chaque extrémité dans une aiguille ;,
- la Figure 4B est une vue de face de la plaque herniaire illustrée à la figure 4A;
- la Figure 5A est une vue de face d'un ballonnet utilisable dans une plaque conforme au premier mode de réalisation de l'invention;
- la Figure 5B est une vue de côté du ballonnet illustré à la figure 5A;
- la Figure 6A est une vue de face d'un autre ballonnet utilisable dans une plaque conforme au premier mode de réalisation de l'invention;
- la Figure 6B est une vue de côté du ballonnet illustré à la figure 6A;
- les Figures 7 à 17 sont des vues de face d'autres ballonnets utilisables dans une plaque conforme au premier mode de réalisation de l'invention;
- les Figures 18 à 27 sont des vues de face de fils ressorts utilisables dans une plaque conforme à un second mode de réalisation de l'invention;
- les Figures 28A à 28D sont des vues en perspective représentant des phases successives d'insertion, dans un canal herniaire, d'une plaque conforme au premier mode de réalisation de l'invention; et
- la Figure 29 est une vue en perspective représentant l'extraction d'un fil ressort hors d'une plaque conforme au second mode de réalisation de l'invention, déployée derrière un canal herniaire.

Comme annoncé précédemment, l'invention concerne une plaque herniaire, c'est-à-dire une plaque destinée à pallier une faiblesse d'une paroi du corps humain en se substituant à elle pour assurer une fonction de maintien.

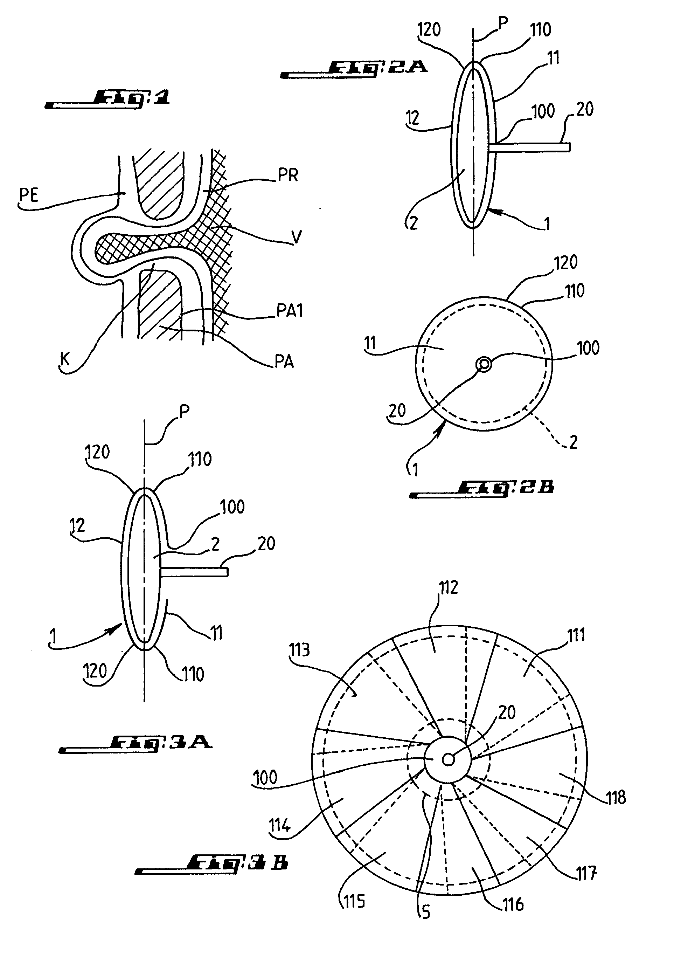

La figure 1 représente une hernie de la paroi abdominale PA, cette dernière étant formée de muscle et de graisse.

Cette pathologie se traduit par la formation d'un canal herniaire K, à travers lequel passent le péritoine PR et des viscères V, qui forment ensemble une excroissance sous la peau PE.

La plaque herniaire, qui vient se placer entre la face interne PA1 de la paroi abdominale PA et le péritoine PR, a ainsi pour rôle, de façon connue en soi, de boucher le canal herniaire K.

La plaque de l'invention comprend essentiellement une poche 1, qui est formée de deux couches textiles 11 et 12, et des moyens d'expansion, tels qu'un ballonnet 2 ou un fil ressort 3, pour assurer un déploiement de la poche 1 derrière la face interne PA1 de la paroi abdominale PA.

Les couches textiles 11 et 12 sont liées l'une à l'autre par leurs périphéries respectives 110 et 120.

Par exemple, les couches textiles 11 et 12 peuvent être réunies l'une à l'autre par couture, soudure ou collage (figures 2A et 2B), ou appartenir à une même bande de textile (figures 3A et 3B).

Selon l'invention, les moyens d'expansion, tels que le ballonnet 2 ou le fil ressort 3, sont reçus de façon amovible dans la poche 1.

Pour ce faire, la couche textile 11 de la poche 1, qui est destinée à venir sur la face interne PA1 de la paroi abdominale PA, présente un orifice 100 qui permet le retrait de ces moyens d'expansion 2 ou 3 hors de la poche 1, après le déploiement de celle-ci derrière le canal herniaire K.

Par ailleurs, quelle que soit leur forme particulière de réalisation, les moyens d'expansion 2 ou 3 passent par déformation réversible d'une configuration déployée à une configuration ramassée en boule.

Dans ces conditions, la plaque, considérée dans son ensemble, c'est-à-dire comprenant la poche textile 1 et un moyen d'expansion tel qu'un ballonnet 2 ou un fil ressort 3, peut elle-même adopter une configuration en boule, ce qui permet son insertion aisée dans le canal herniaire K.

Dans le cas général, la couche textile 12, c'est-à-dire la couche textile destinée à venir se placer contre le péritoine PR, peut être conformée en disque, et l'orifice 100 adopte de préférence une position centrale par rapport à la poche 1.

Si les couches 11 et 12 sont formées dans une même bande textile, comme illustré aux figures 3A et 3B, la couche textile 11 est avantageusement découpée en plusieurs secteurs radiaux tels que 111 à 118, qui sont distribués autour de l'orifice 100 et qui se recouvrent mutuellement. Ces secteurs radiaux peuvent être fixés par un lien coulissant circulaire, qui en assure la liaison.

Les figures 2A à 17 illustrent l'utilisation d'un ballonnet 2 en tant que moyen d'expansion.

Ce ballonnet, qui est étanche au gaz et / ou à un liquide plus ou moins radio-opaque, est équipé d'un conduit plus ou moins rigide 20 de gonflage, prévu pour traverser l'orifice 100 de la couche textile 11.

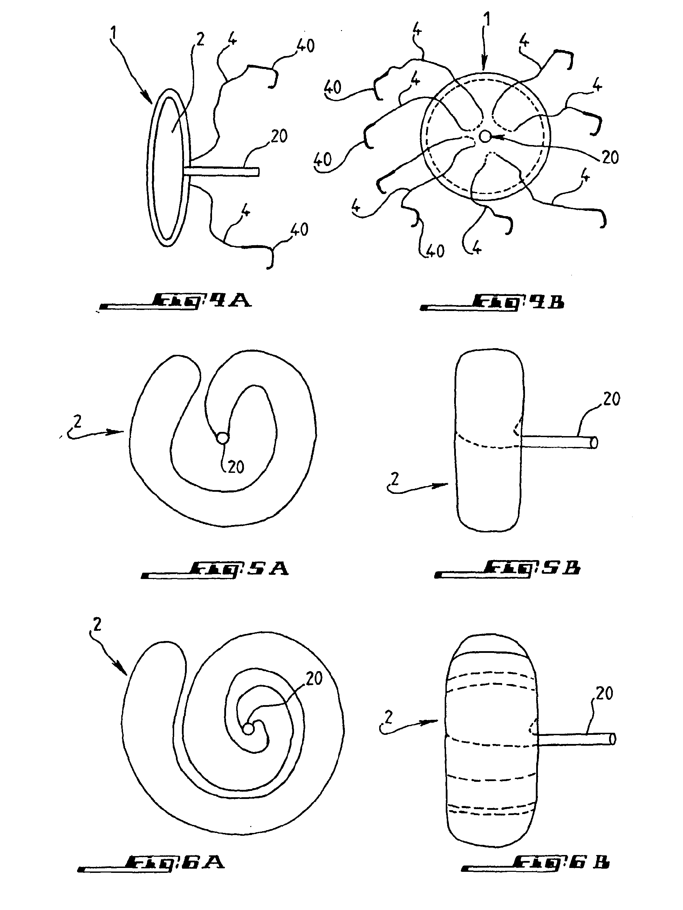

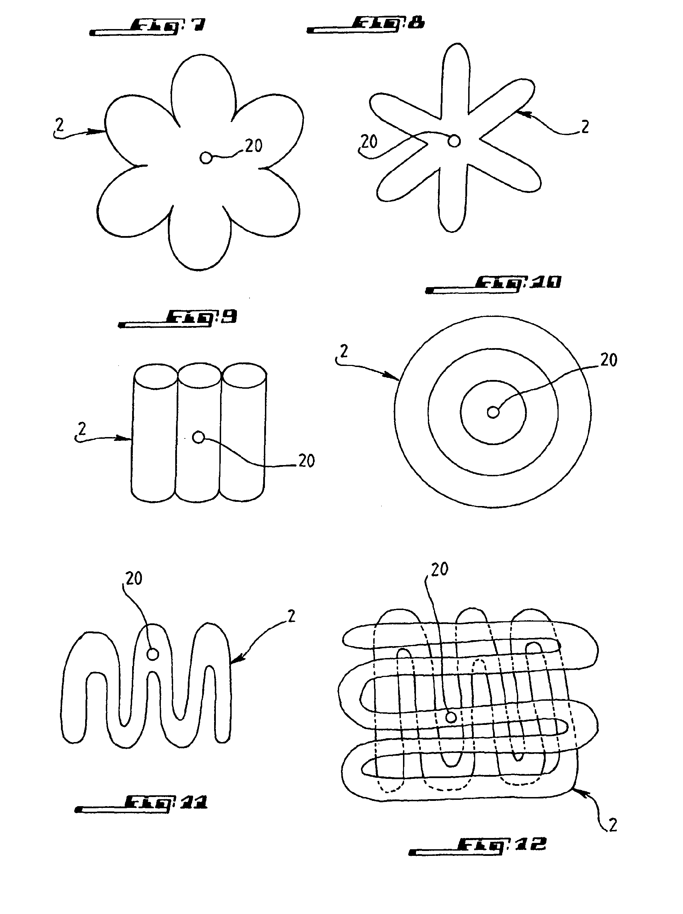

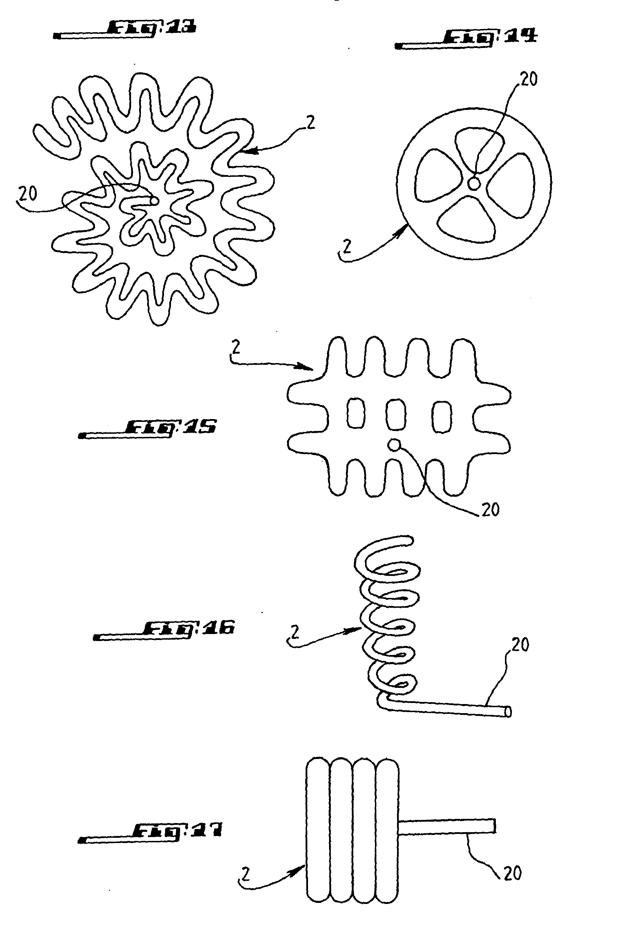

En configuration déployée, le ballonnet 2 peut adopter une forme choisie dans un grand nombre de formes possibles, et être notamment conformé en spirale plus ou moins développée (figures 5A à 6B), en fleur (figure 7), en étoile (figure 8), en matelas pneumatique (figure 9), en tore simple ou multiple (figure 10), en serpentin simple (figure 11), en serpentin double à couches croisées (figure 12), en serpentin spiralé (figure 13), en roue (figure 14), ou en grille (figure 15).

Dans tous les cas cités, le ballonnet 2 s'étend principalement dans un plan médian P, c'est-à-dire que les deux dimensions privilégiées qui constituent sa longueur et sa largeur forment un plan médian P dont le ballonnet 2 s'écarte peu en hauteur.

Néanmoins, le ballonnet 2 peut s'étendre davantage en volume et présenter, en configuration déployée, une forme en hélice (figure 16) ou une forme en accordéon (figure 17).

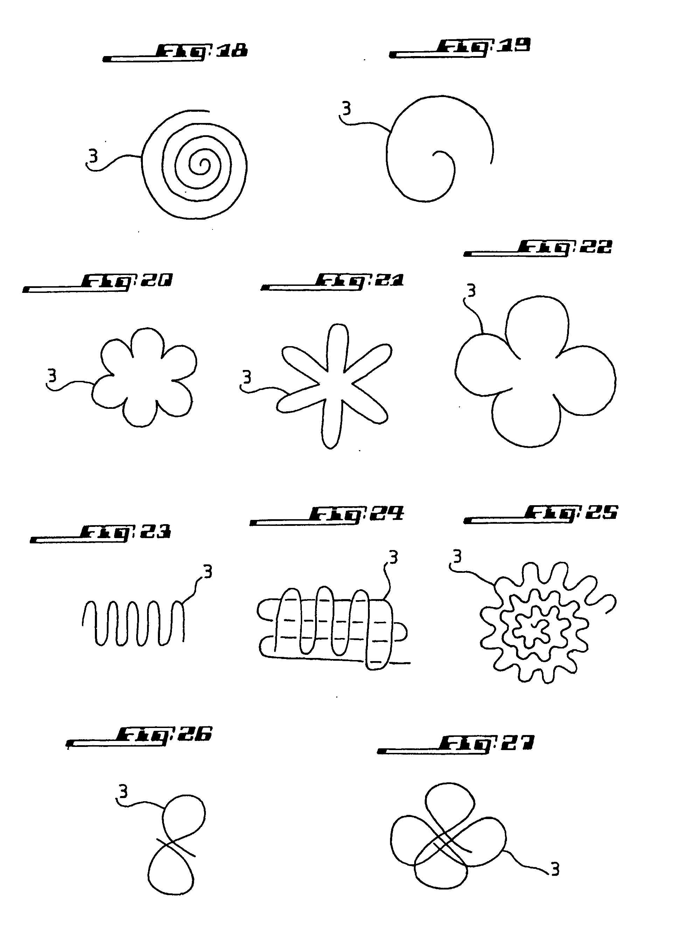

Les figures 18 à 27 illustrent l'utilisation d'un fil ressort 3 en tant que moyen d'expansion, ce fil 3 étant de préférence constitué par un fil métallique monofilament.

En configuration déployée, le fil ressort 3 peut lui aussi adopter une forme choisie dans un grand nombre de formes possibles, et être notamment conformé en spirale plus ou moins développée (figures 18 et 19), en fleur ou en étoile (figures 20 à 22), en serpentin simple (figure 23), en serpentin double à couches croisées (figure 24), en serpentin spiralé (figure 25), en huit simple (figure 26), ou en huit multiple, par exemple en double huit (figure 27).

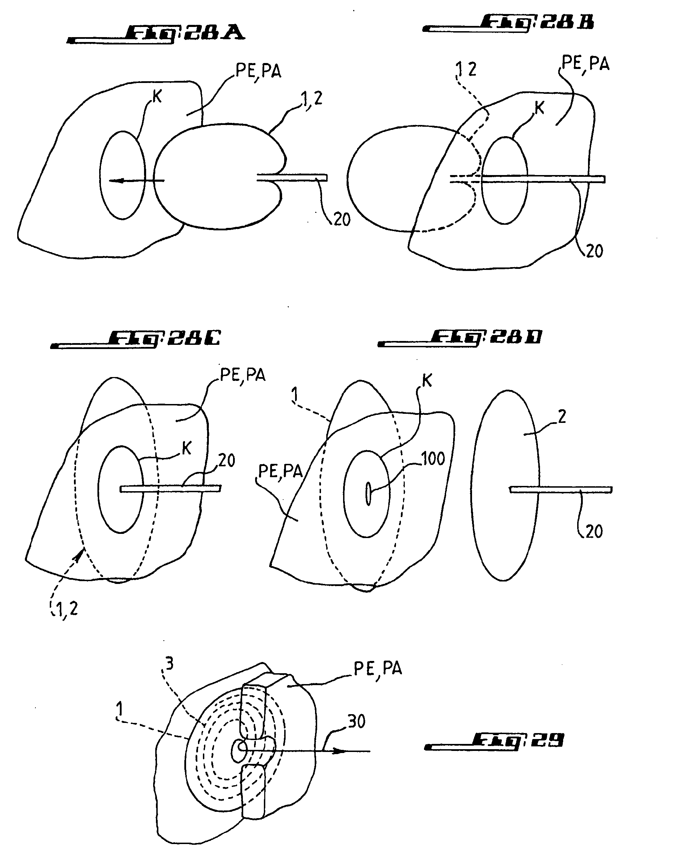

Les figures 28A à 28D illustrent l'utilisation d'une plaque herniaire conforme à l'invention, cette plaque étant en l'occurrence supposée incorporer un ballonnet 2.

Tout d'abord (figure 28A), la plaque formée par la poche 1 et le ballonnet 2 est repliée en boule autour du conduit de gonflage 20 et poussée dans le canal herniaire K jusqu'à s'introduire dans ce dernier jusqu'à son orifice profond.

Après introduction complète de la plaque 1, 2 dans le canal K, le ballonnet 2 est rempli d'air ou d'un liquide plus ou moins radio-opaque à travers le conduit 20, de sorte qu'il se gonfle et qu'il étale la poche 1 entre le péritoine PR et la face interne PA1 de la paroi abdominale PA (figure 28C).

Une fois que la poche 1 est en place, le ballonnet 2 peut être dégonflé, et extrait de la poche 1 et du canal herniaire K (figure 28D).

Dans le cas où la plaque de l'invention utilise un fil ressort 3 en tant que moyen d'expansion, cette plaque est mise en place comme dans le cas précédent.

En revanche, l'extraction du fil ressort 3 après la pose de la poche 1 peut justifier de prévoir que ce fil 3 présente un brin 30 accessible au travers de l'orifice 100 de la couche textile 11, de manière que ce fil 3 puisse être extrait de la poche 1 par traction sur ce brin 30.

Dans tous les cas, et en particulier dans ce dernier cas, la plaque de l'invention peut comprendre, comme le montrent les figures 4A et 4B, des fils de suture 4 solidaires de la couche textile 11, sertis dans des aiguilles 40 à chacune de leurs extrémités, et permettant de lier la poche 1 à la paroi abdominale PA, après déploiement de cette poche derrière la face interne PA1 de la paroi abdominale PA.

## Revendications

1. Plaque herniaire, comprenant une poche (1) formée de première et seconde couches textiles (11, 12) liées l'une à l'autre par leurs périphéries respectives (110, 120), et des moyens d'expansion (2, 3) assurant un déploiement de la poche (1) derrière la face interne (PA1) d'une paroi musculaire (PA), les moyens d'expansion (2, 3) Etant reçus de façon amovible dans la poche (1), la première couche textile (11) présentant un orifice (100) permettant le retrait des moyens d'expansion (2, 3) hors de la poche (1) après le déploiement de celle-ci, et
les moyens d'expansion (2, 3) passant par déformation réversible d'une configuration déployée à une configuration ramassée en boule, ce dont il résulte que la plaque dans son ensemble (1, 2; 1, 3) peut elle-même adopter une configuration en boule pour permettre son insertion dans le canal herniaire (K), **caractérisée en ce que** l'orifice (100) a une position centrale par rapport à la poche (1) et **en ce que** la première couche textile (11) est découpée en plusieurs secteurs radiaux (111, 112, 113) distribués autour de l'orifice (100) et se recouvrant, mutuellement.

2. Plaque herniaire suivant la revendication 1, **caractérisée en ce que** la seconde couche textile (12) est conformée en disque.

3. Plaque herniaire suivant la revendication 1, **caractérisée en ce qu'**elle comprend un lien coulissant circulaire (5) assurant la liaison des secteurs radiaux (111, 112, 113).

4. Plaque herniaire suivant l'une quelconque des revendications 1 à 3, **caractérisée en ce que** les moyens
d'expansion (2, 3) comprennent un ballonnet (2) étanche au gaz et / ou à un liquide, ce ballonnet (2) comprenant un conduit plus ou moins rigide (20) de gonflage, traversant l'orifice (100) de la première couche (11).

5. Plaque herniaire suivant la revendication 4, **caractérisée en ce que** le ballonnet (2), en configuration déployée, s'étend dans un plan médian (P).

6. Plaque herniaire suivant la revendication 5, **caractérisée en ce que** le ballonnet (2), en configuration déployée, est conformé en étoile, en fleur, en spirale, en serpentin, en grille, en matelas, en roue, ou en tore simple ou multiple.

7. Plaque herniaire suivant la revendication 4, **caractérisée en ce que** le ballonnet (2), en configuration déployée, est conformé en hélice ou en accordéon.

8. Plaque herniaire suivant l'une quelconque des revendications 1 à 3, **caractérisée en ce que** les moyens d'expansion (2, 3) comprennent un fil ressort (3).

9. Plaque herniaire suivant la revendication 8, **caractérisée en ce que** le fil ressort (3) est un fil métallique monofilament.

10. Plaque herniaire suivant la revendication 9, **caractérisée en ce que** le fil ressort (3), en configuration déployée, est conformé en étoile, en fleur, en spirale, en serpentin, ou en huit simple ou multiple.

11. Plaque herniaire suivant les revendications 9 ou 10, **caractérisée en ce que** le fil ressort (3) est extractible de la poche (1) par traction sur un brin (30) du fil ressort accessible à travers l'orifice (100).

12. Plaque herniaire suivant l'une quelconque des revendications précédentes, **caractérisée en ce qu'**elle comprend des fils de suture (4) solidaires de la première couche textile (11).

## Claims

1. A hernia plate, comprising a pocket (1) formed by first and second textile layers (11, 12) connected to each other by their respective peripheries (110, 120), and expansion means (2, 3) ensuring deployment of the pocket (1) behind the inner surface (PA1) of a muscle wall (PA), the expansion means (2, 3) being removably received in the pocket (1), the first textile layer (11) having an orifice (100) allowing the withdrawal of the expansion means (2, 3) outside the pocket (1) after the deployment thereof, and the expansion means (2, 3) passing through reversible deformation from a deployed configuration to a compact ball configuration, from which it results that the plate in its entirety (1, 2; 1, 3) can in turn adopt a ball configuration to allow it to be inserted into the hernial channel (K), **characterized in that** the orifice (100) has a central position relative to the pocket (1) and **in that** the first textile layer (11) is cut into several radial sectors (111, 112, 113) distributed around the orifice (100) and overlapping.

2. The hernia plate according to claim 1, **characterized in that** the second textile layer (12) is shaped in a disk.

3. The hernia plate according to claim 1, **characterized in that** it comprises a circular sliding link (5) ensuring the connection of the radial sectors (111, 112, 113).

4. The hernia plate according to any one of claims 1 to 3, **characterized in that** the expansion means (2, 3) comprise a gas- and/or liquid-tight cuff (2), said cuff (2) comprising a more or less rigid inflating pipe (20), passing through the orifice (100) of the first layer (11).

5. The hernia plate according to claim 4, **characterized in that** the cuff (2), in the deployed configuration, extends in a middle plane (P).

6. The hernia plate according to claim 5, **characterized in that** the cuff (2), in the deployed configuration, is shaped as a single or multiple star, flower, spiral, coil, grid, cushion, wheel, or toroid.

7. The hernia plate according to claim 4, **characterized in that** the cuff (2), in the deployed configuration, is configured in a helix or accordion.

8. The hernia plate according to any one of claims 1 to 3, **characterized in that** the expansion means (2, 3) comprise a spring wire (3).

9. The hernia plate according to claim 8, **characterized in that** the spring wire (3) is a single-filament metal wire.

10. The hernia plate according to claim 9, **characterized in that** the spring wire (3), in the deployed configuration, is shaped in a single or multiple star, flower, spiral, coil, or eight.

11. The hernia plate according to claims 9 or 10, **characterized in that** the spring wire (3) can be removed from the pocket (1) by pulling on a strand (30) of the spring wire accessible through the orifice (100).

12. The hernia plate according to any one of the preceding claims, **characterized in that** it comprises suture threads (4) secured to the first textile layer (11).

## Patentansprüche

1. Hernienplatte, die eine Tasche (1) umfasst, die von einer ersten und zweiten Textilschicht (11, 12) gebildet wird, die durch ihre jeweiligen Peripherien (110, 120) miteinander verbunden sind, und Expansionsmittel (2, 3), die ein Entfalten der Tasche (1) hinter der Innenseite (PA1) einer Muskelwand (PA) sicherstellen, wobei die Expansionsmittel entfernbar in der Tasche (1) aufgenommen sind, wobei die erste Textilschicht (11) eine Öffnung (100) aufweist, die die Entfernung der Expansionsmittel (2, 3) aus der Tasche (1) nach dem Entfalten derselben erlauben, und Expansionsmittel (2, 3), die durch umkehrbare Verformung aus einer entfalteten Konfiguration in eine kugelförmig komprimierte Konfiguration übergeben, woraus sich ergibt, dass die Platte insgesamt (1, 2; 1, 3) eine kugelförmige Konfiguration annehmen kann, um ihr Einsetzen in den Hernienkanal (K) zu erlauben, **dadurch gekennzeichnet, dass** die Öffnung (100) im Verhältnis zur Tasche (1) eine zentrale Stellung hat und dass die erste Textilschicht (11) in mehrere radiale Sektoren (111, 112, 113) unterteilt ist, die um die Öffnung (100) verteilt sind und sich gegenseitig überlappen.

2. Hernienplatte nach Anspruch 1, **dadurch gekennzeichnet, dass** die zweite Textilschicht (12) scheibenförmig ausgebildet ist.

3. Hernienplatte nach Anspruch 1, **dadurch gekennzeichnet, dass** sie eine kreisförmige gleitende Verbindung (5) umfasst, die die Verbindung der radialen Sektoren (111, 112, 113) sicherstellt.

4. Hernienplatte nach einem der Ansprüche 1 bis 3, **dadurch gekennzeichnet, dass** die Expansionsmittel (2, 3) einen gas- und/oder flüssigkeitsdichten Ballon umfassen, wobei dieser Ballon (2) eine mehr oder weniger starre Leitung (20) zum Aufblasen umfasst, die die Öffnung (100) der ersten Schicht (111) durchquert.

5. Hernienplatte nach Anspruch 4, **dadurch gekennzeichnet, dass** sich der Ballon (2) in entfalteter Konfiguration in einer mittleren Ebene (P) erstreckt.

6. Hernienplatte nach Anspruch 5, **dadurch gekennzeichnet, dass** der Ballon (2) in entfalteter Konfiguration als Stern, Blüte, Spirale, Serpentine, Gitter, Polster, Rad oder als einfacher oder vielfacher Torus ausgebildet ist.

7. Hernienplatte nach Anspruch 4, **dadurch gekennzeichnet, dass** der Ballon (2) in entfalteter Konfiguration als Schraube oder Ziehharmonika ausgebildet ist.

8. Hernienplatte nach einem der Ansprüche 1 bis 3, **dadurch gekennzeichnet, dass** die Expansionsmittel (2, 3) einen Federdraht (3) umfassen.

9. Hernienplatte nach Anspruch 8, **dadurch gekennzeichnet, dass** der Federdraht (3) ein Monofil-Metalldraht ist.

10. Hernienplatte nach Anspruch 9, **dadurch gekennzeichnet, dass** der Federdraht (3) in entfalteter Konfiguration als Stern, Blüte, Spirale, Serpentine oder einfache oder vielfache Acht ausgebildet ist.

11. Hernienplatte nach den Ansprüchen 9 oder 10, **dadurch gekennzeichnet, dass** der Federdraht (3) aus der Tasche (1) durch Zug an einem Faden (30) des Federdrahts entfernbar ist, der durch die Öffnung (100) erreichbar ist.

12. Hernienplatte nach einem der vorangehenden Ansprüche, **dadurch gekennzeichnet, dass** sie Nähfäden (4) umfasst, die mit der ersten Textilschicht (11) verbunden sind.
